# EUROPEAN PATENT APPLICATION

(11) **EP 0 780 478 A1**
(43) Date of publication of application: **25.06.1997**
(21) Application number: 95402910.4
(22) Date of filing: 21.12.1995
(51) Int. Cl.: C12Q 1/68

(54) **Method for the indirect genotypic diagnosis of cadasil**

(71) Applicant: INSTITUT NATIONAL DE LA SANTE ET DE LA RECHERCHE MEDICALE (INSERM), 75654 Paris Cédex 13 (FR); ASSISTANCE PUBLIQUE, 75100 Paris (FR)
(72) Inventor: Tournier-Lasserve, Elisabeth, 75016 Paris (FR); Joutel, Anne, 75005 Paris (FR); Bousser, Marie-Germaine, 75014 Paris (FR); Bach, Jean-Francois, 75015 Paris (FR)
(74) Representative: Polus, Camille

(57) **Abstract**

The method for the indirect genotypic diagnosis of CADASIL for symptomatic or at risk individuals or fetuses belonging to a family suspected or known to be affected by CADASIL, comprises the use of markers genetically linked to the mutated gene responsible for CADASIL in order to detect whether or not the tested individual is carrying the chromosome 19 markers alleles that has been linked to the disease gene in this given family and to estimate his carrier risk, the method being based on the localization of the gene in the interval of 2 cM spanned by the flanking markers D19S226 and D19S199 and in that one uses at least two markers located each on one side of the gene.

## Description

The present invention relates to a method for the indirect genotypic diagnosis of Cerebral Autosomal Dominant Arteriopathy with Subcortical Infarts and Leukoencephalopathy (CADASIL).

### Background of the Invention

Stroke is the third leading cause of death and the first cause of acquired physical or cognitive impairment in developed countries. Strokes are ischaemic in 80 % of cases and the leading causes are atheroma and cardiac emboli. But despite extensive investigation, up to 40 % of cases remain without definite aetiology.

Familial causes of stroke have recently been identified, such as MELAS and homocystinuria. Since 1977, nine unrelated families have been reported with a new mendelian syndrome that leads to stroke. Recently was reported a pedigree, the large size of which allowed the precise definition of the clinical, neuro-imaging and genetic parameters of this disease (Tournier-Lasserve et al, Stroke, 1991, 22, 1297-1302, Tournier-Lasserve et al, Nature Genetics, 1993, 3, 256-259). This condition is characterized by recurrent subcortical ischaemic strokes and dementia. It is underlaid by a cerebral non- atherosclerotic, non-amyloid angiopathy affecting mainly the small arteries penetrating the white matter and basal ganglia. All reported families share strikingly similar clinical, neuro-imaging and pathological features. The acronym CADASIL (cerebral autosomal dominant arteriopathy with subcortical infarcts and leukoencephalopathy) is used.

Genetic linkage analysis conducted on 2 large CADASIL pedigrees assigned the CADASIL locus to chromosome 19 and multilocus analysis with the location scores method established the best estimate for the location of the gene within a 14 cM interval bracketed by D19S221 and D19S215 loci (E. Tournier-Lasserve et al., Nature Genetics, 1993, 3, 256-259 and A. JOUTEL et al., Nature Genetics, 1993, 5, 40-46).

The first publication indicates a number of microsatellites useful for linkage analysis : D19S216, D19S221, D19S226, D19S222, D19S208.

All CADASIL families tested until now are genetically homogenous and map to the same previously defined interval on chromosome 19.

The reliability of indirect genetic linkage genotyping is increased when using markers located very close to the gene and on both sides of this gene since the use of such markers will decrease the error risk due to potential double recombinants

Up to now, the size of the interval containing the CADASIL gene was 14 cM, this interval being flanked by D19S221 and D19S215. The risk for an individual to be double recombinant for these 2 markers could be estimated to be approximately 0.07 x 0.07 = 0.0049 ≃ 0.5 % meaning that the error risk when genotyping one individual was 0.5 %. In order to reduce this error risk, one needs to reduce the size of the interval containing the gene.

Therefore, the need exists always to further reduce the genetic interval comprising the gene responsible for CADASIL and to propose a very accurate diagnosis method.

### Summary of the invention

After extensive researches and studies on a number of new CADASIL families, the Applicant succeeded in greatly shortening the interval in which the gene responsible for CADASIL is located to an interval of 2 cM. This allows one to propose an indirect genotypic diagnosis of high accuracy and safety by using specifically the microsatellites flanking and spanning this 2 cM interval. The risk for a double recombinant within this interval being now 0.01 x 0.01 = 10⁻⁴ as compared to 50.10⁻⁴ previously. In addition, the microsatellite D19S253-D19S244 and two new microsatellites D19S841 and 11547 have been identified and mapped within the interval. These very highly polymorphic markers are located very close to the gene within this interval. Their use in the diagnosis method according to the invention will further increase its accuracy and safety.

The invention has several objects :
- One object of the invention is to provide a method for diagnosing CADASIL within a family suspected to be affected by CADASIL.
- Another object of the invention is to provide a method to identify the CADASIL families to be analyzed for predictive testing by genetic linkage analysis with chromosome 19 markers.
- It is yet another object of the invention to provide a method for predictive testing an "at risk" individual within a CADASIL family.
- Another object of the invention is to provide a method for prenatal diagnosis of CADASIL.

The object of the invention is thus a method for the indirect genotypic diagnosis of CADASIL for symptomatic or at risk individuals or fetuses belonging to a family suspected or known to be affected by CADASIL, the method comprising the use of markers genetically linked to the mutated gene responsible for CADASIL in order to detect whether or not the tested individual is carrying the chromosome 19 markers alleles that have been linked to the disease gene, also called the "affected" alleles of the markers, in this given family, and to estimate his carrier risk, characterized in that the method is based on the localization of the gene in the interval of 2 cM spanned by the flanking markers D19S226 and D19S199 and in that one uses at least two markers located on both sides of the gene. This localization allows one to calculate the error risk related to a double recombination within the interval with a very high accuracy, taking into account the very small size of the interval.

According to the preferred embodiment of the invention corresponding to the use of at least two markers selected from the group of the so-called flanking markers and the markers located within the 2 cM interval, the method according to the invention is a method allowing to diagnose the CADASIL status or the healthy status with a percentage of error risk estimated to be below 10⁻⁴.

Preferably, one uses at least one of the markers selected from the following microsatellites : D19S226, D19S199, D19S841, 11547, D19S253 and D19S244.

D19S226 and D19S199 are the microsatellites flanking the 2 cM interval in which the gene responsible for CADASIL is located. D 19S841, 11547, D19S253, D19S244 and D19S411 have been mapped within this interval. These microsatellites are very close to the gene and highly informative.

In one appropriate embodiment of the invention, one uses both markers D19S226 and D19S199. The interval between these markers is approximately 2 cM and the use of both markers allows one to conclude about the status of a family (linked or not to the mutated gene) and the carrier risk. In this case, the percentage of error risk is of the order of 1/10,000, which is a very high accuracy.

However, it can not be excluded that one of these microsatellites, e.g. D19S199 or D19S226, is not informative for one family or one individual. In that case, one may choose another marker located further than the non-informative marker from the gene but on the same side with respect to the gene. The knowledge of the genetic distance of this marker with respect to the informative marker located on the other side of the gene and used therewith allows one to calculate the accuracy of the diagnosis.

So, according to the invention, one may also use another markers selected from the group consisting of the following microsatellites : D19S221, D19S179, D19S252, D19S415 and D19S215.

D19S841 and 11547 are located within the interval of 2 cM and may be used according to one appropriate embodiment, alone or together, with the surrounding markers to increase the safety and accuracy of the diagnosis, say in order to eliminate the presence of a double recombination.

Each of the microsatellites D19S199, D19S226, D19S841 and 11547 may be used in combination with a marker located on the other side of the gene location on the chromosome.

Another useful marker are the microsatellites D19S253, D19S244 located within the 2cM interval. These microsatellites may be used as the third marker or as a flanking marker together with another marker located on the other side of the gene on the chromosome, for example 11547.

As it is well known, the calculation of the risk of recombination is dependant from the genetic distance. By using two flanking markers, the risk is calculated on this basis : for a 1 cM genetic interval between both markers : 0.005 x 0.005. For example with the 2 cM interval, the risk is : 0.01 x 0.01 = 0.0001.

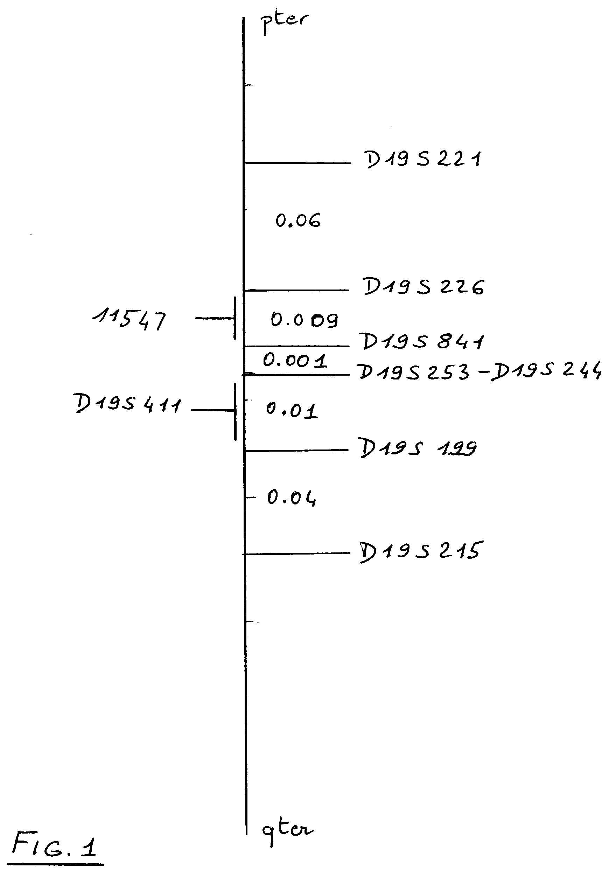

To know the genetic distances of the different microsatellites discussed above, reference is made to the attached figure 1.

All known polymorphic markers flanking D19S226 on the telomeric side and D19S199 on the centromeric side may be used as flanking marker. Also, all known polymorphic markers located in the interval may be used as third marker. The choice may be made in the genome data bank.

Linkage analysis is conducted on families which structure is suitable for such analysis, namely families comprising multiple individuals whose clinical and cerebral magnetic resonance imaging (MRI) status (healthy or affected) has been unambiguously established (see Nature Genetics, 1993, 3, 256-259). Disease status for linkage analysis is based on the cerebral MRI data. All clinically affected members have an abnormal cerebral MRI. At risk aymptomatic individuals whose MRI shows the same lesions as the ones observed in clinically affected subjects are considered as affected. Asymptomatic offspring of an affected individual having a normal MRI is considered as having an unknown status when aged below 35 years old.

Preferably, the method comprises the use of primer pairs which hybridize specifically on DNA sequences flanking the marker alleles which are known to be linked to the mutated gene responsible for CADASIL by ill individuals, the amplification of the DNA to be tested in the presence of the primer pairs and the analyzis of the amplified products to assess the presence or the absence of markers alleles linked to the mutated gene, and the estimation of the carrier risk of the tested individual with respect to these allele markers.

The microsatellites are described in the microsatellites Genethon map (Weissenbach J. & al., Nature 359, 794-801, 1992, Gyapay et al. 1994,. The 1993-1994 Genethon human genetic linkage map, Nat. Genet. 7 : 246-339, Hudson et al. 1992, Genomics 13 : 622-629 and Weber and May 1989....or in J. Weber et al, Am. J. Hum. Gen. 1993, 53, 1079-1095. Microsatellite D19S841 and 11547 are described in the enclosed detailed specification.

The oligonucleotide sequences serving as primers, which are specific of each microsatellite except those of D19S841 and 11547 are available in the Genome Data Bank (Accessing GDB™ and OMIM™ at the John Hopkins University, Baltimore, Maryland, USA). The sequences for D19S841 and 11547 are given in the detailed specification.

The preferred method consists of hybridization of the primers to the DNA to be tested, followed by DNA amplification by Polymerase Chain Reaction amplification.

Preferably, the polymorphic amplified fragments, so-called amplimers, are separated after amplification according to their size by electrophoresis on acrylamide denaturing gels, blotted on nylon membranes and hybridized with CA12mer or GATA 5mer radiolabelled probes. Data obtained from autoradiographies are computed and Lod-score calculations are carried out using the M-LINK program (Lathrop et al, P.N.A.S., 1984, 81, 3443-3446 incorporated therein by reference). Based on MRI data, penetrance has been established to be complete after 35 years old (see above).

The assertion of linkage or absence of linkage is derived from well-established statistical analysis, a lod-score above 3 establishing unambiguous linkage of the disease gene to the tested marker. A lod-score value of 3 is in fact needed only when the prior probability of linkage between a disease gene and a marker is no more than random, which is not anymore the case for CADASIL. The strictness of this criterion would be soon decreased according to the prior probability values observed in on going epidemiological studies.

Another object of the invention is the nucleotide sequences of the microsatellites D19S841 and 11547 delimited by the hereinafter cited oligonucleotide sequences serving as primers for these microsatellites, as well as oligonucleotide sequences comprising these oligonucleotides and useful as primers.

For amplification, the oligonucleotides have generally from about 15 to about 60 nucleotides, preferably 20 to 25, and comprise the sequences flanking the markers D19S841 and 11547 which are disclosed hereinafter under SEQ ID NO: 1 to 4.

Of course, the invention includes also the sequences having some variations in the nucleotides which do not affect the specific hybridization between primer and corresponding DNA.

### Brief description of the figures :

**Figure 1 : Schematic regional map of chromosome 19p** showing the main markers according to the invention with the genetic distance in cM. The location of D19S411 and 11547 is indicated on the left.

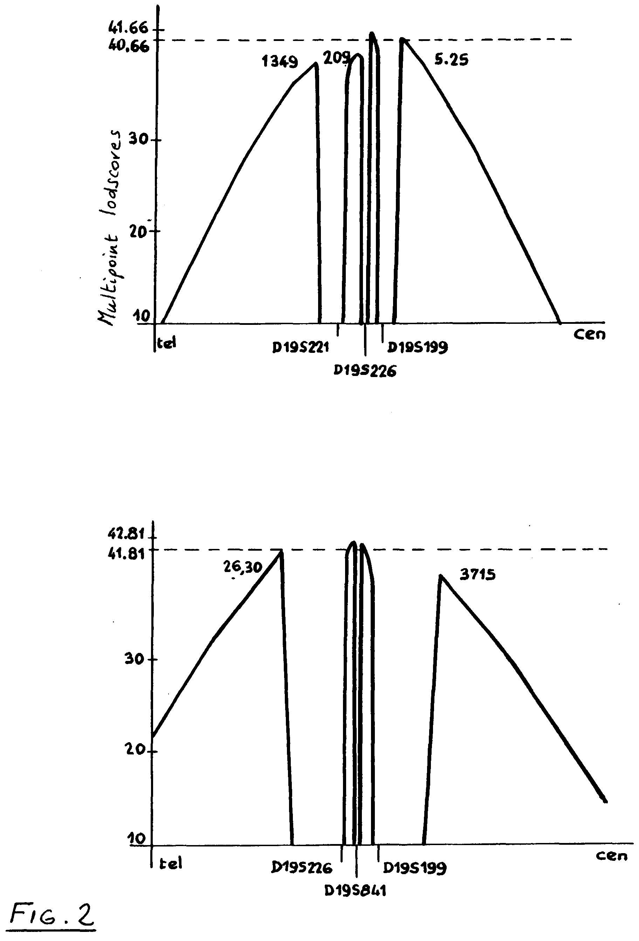

**Figure 2 : Multipoint analysis.** Multipoint Lod scores for different positions of the CADASIL locus are shown with respect to two subsets of 3 markers, on the left panel : D19S221-(0.06)-D19S226-(0.02)-D19S199, on the right panel : D19S226-(0.009)-D19S841-(0.011)-D19S199. The solid lines indicate the 10:1 odds (1-lod unit) interval for the placement of the gene. Odds against alternatives are shown for the most likely placement of the CADASIL locus in each interval.

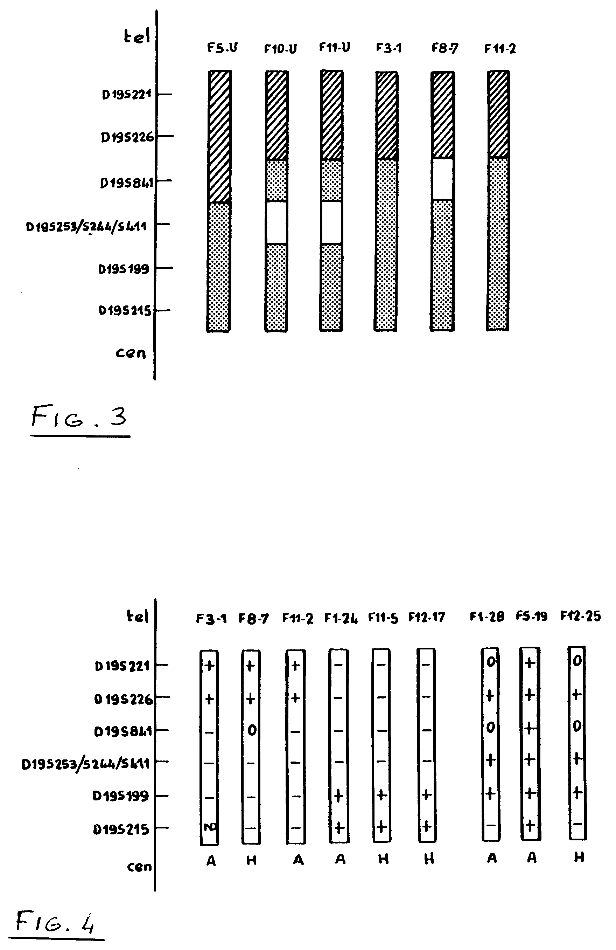

**Figure 3 :**Crossover events observed within the D19S221-D19S215 interval. Haplotype analysis of the 15 families revealed six crossover events that led to refine the position of marker D19S411, previously mapped at θ = 0 from D19S226, and to precisely locate the new marker D19S841. A schematic diagram of the relevant region on 19p is shown on the left (not to scale). Because no recombination event was observed between markers D19S253, D19S244 and D19S411, they are represented as a single locus. For each individual in whom haplotype analysis revealed a crossover event, the involved chromosome is represented by a bar. Above the bar, the family and the position in the pedigree of this individual are indicated. Three crossover events were observed in individuals of unknown status (F5-U, F10-U, and F11-U) observed in individuals of unknown status (F5-U, F10-U, and F1-U) on the chromosome inherited from their unaffected parent. Tinted and hachured parties = haplotypes transmitted by a given parent and carried by his homologous chromosomes. White parts = uninformative in this region.
**Figure 4 :** Recombination events leading to the precise mapping of the CADASIL locus. For each individual in whom haplotype analysis revealed a recombination event, the chromosome inherited from the affected parent is represented by a bar. A schematic map of 19p (not to scale) is shown on the left. Because no crossover event occured between markers D19S253, D19S244, and D19S411, the latter are represented as a single locus. A = affected ; H = healthy ; plus sign (+) = recombinant ; minus sign (-) = nonrecombinant ; 0 = uninformative and ND = nor done.

### Detailed specification

### Families and status definition :

A total of 15 unrelated families from five different european countries were genotyped. Two of them, F1 and F2, have already been genotyped with several chromosome 19 markers and were further investigated with new markers (Tournier-Lasserve et al. 1993).

Thirteen additional families were analyzed. They have all been selected on the basis of the following criteria : (1) an history of recurrent strokes without any vascular risk factors for the propositus and at least one relative, (2) a leukoaraiosis with or without small deep infarcts on brain MRI in all clinically affected patients, (3) a pattern of inheritance consistent with an autosomal dominant mode.

Fifty such families were referred : 13 included at least 6 potentially informative meiosis ; they were therefore considered as potentially informative and genotyped. Two hundred and twenty eight subjects (> 18 years old) from these 15 families gave their informed consent for this study. They were examined by a board certified neurologist, underwent cerebral MRI and were blood drawn.

As described previously, MRI was used to establish the status for genetic linkage analysis (Tournier-Lasserve et al. 1993). Briefly, individuals with an abnormal MRI were considered as affected, whether or not clinically symptomatic ; asymptomatic individuals with a normal MRI were considered as healthy when aged above 35, and as having an unknown status when younger than 35.

One hundred and fourteen individuals had an abnormal MRI : among them, seventy-one members experienced neurological symptoms and 43 were totally asymptomatic. Neurological symptoms included transient ischemic attacks or completed strokes (51 patients), migraine with aura (26 patients, including 15 patients suffering from both strokes and migraine with aura and 11 patients suffering only from migraine with aura), dementia (22 patients with preceding strokes and 6 patients with an isolated progressive dementia), mood disorders (8 patients with associated strokes and 3 patients with isolated mood disorders).

One hundred and fourteen individuals had an MRI showing neither White Matter Abnormalities nor subcortical infarcts : 85 of them aged above 35 years were classified as healthy, 29 of them were younger than 35 and classified as having an unknown status. Among the 85 patients classified as healthy, two individuals had an MRI showing sequelae from large vessel cerebral infarcts due to atherosclerosis and 2 patients suffered from migraine with aura.

Pathological data were available in 6 of these families (F1-11, F10-PM, F12-PM, F16-PM, F18-1 and F21-3) and showed in all cases a non atherosclerotic, non amyloid angiopathy affecting the small ateries of the cerebral white matter and the basal ganglia.

Karyotype analysis performed in one index case from 8 families (F1, F2, F5, F8, F10, F11, F13 and F14) was unremarkable.

### Markers :

All 15 families were analysed with eight polymorphic microsatellite markers : D19S221, D19S226, D19S411, D19S215, D19S253, D19S244, D19S199 and D19S841.

The new D19S841 marker is characterized by the flanking nucleotide sequences :
SEQ ID NO : 1 TCCTGAGCTCAGGCAAT
SEQ ID NO : 2 CCAAGCTTTGGAGATGTC

Eight alleles have been found for this new marker:

| Alleles : | Fragment Sizes (kb) : | Frequencies : |
|---|---|---|
| 1 | 0.252 | 0.02 |
| 2 | 0.250 | 0.09 |
| 3 | 0.248 | 0.25 |
| 4 | 0.246 | 0.11 |
| 5 | 0.244 | 0.05 |
| 6 | 0.242 | 0.04 |
| 7 | 0.240 | 0.12 |
| 8 | 0.238 | 0.26 |

PCR informations :
- Amplified product size : 0.252 to 0238 kb
- Final extension temperature : 72°C
   Final extension time : 10 min
- Template concentration : 400 ng/100 µl
- Primer concentration : 1 µM
- dNTP concentration : 125 µM
- Polymerase : 1 U
- Reaction volume : 50 µl
- Thermal Cycler Name : Perkin Elmer PHC 3
- Denaturation temperature : 94°C
   Denaturation time : 1 min
- Annealing temperature : 55°C
   annealing time : 1 min
- Extension temperature : 72°C
   Extension time : 1 min
- Number of cycles : 28

The new 11547 marker is characterized by the flanking nucleotide sequences :
SEQ ID NO : 3 GCA ATT ATA TCT CCC ACT AAT G
SEQ ID NO : 4 GAC AGA AAG AGA CTC TGT CTG

The size of their alleles is comprised between 235 and 290 bp.

The oligonucleotide sequences flanking the other markers and informations related to these sequences are available through the Genome Data Base (John Hopkins University, Baltimore). D19S253 and D19S244 are (GATA)ₙ repeats and the others are (CA)ₙ repeats. A genetic partial map of chromosome 19 is presented on figure 2.

### Genotyping and linkage analysis :

DNA from peripheral blood from all consenting members including 199 potentially informative meiosis, 45 spouses and 29 subjects of unknown status was extracted. For subjects F18-1 and F21-3, DNA was extracted from autopsy material. Polymorphic genomic sequences were amplified by PCR as described previously (Tournier-Lasserve et al, 1993). Linkage analysis was performed using version 5.1 of the LINEAGE program package (Lathrop et al. 1985, Am. J. Hum. Genet. 37, 482-498 incorporated herein by reference) using published alleles frequencies from CEPH pedigrees. The frequency of the D19S841 alleles was determined by genotyping 70 unrelated subjects, including 28 CEPH families founders. CADASIL gene frequency was set at 0.0001. Lod score obtained with markers D19S221 and D19S226 for families F1 and F2 have already been reported (Tournier-Lasserve et al. 1993), and were incorporated here in the combined Lod score calculation.

Haplotype studies were performed on all families and the most likely haplotypes were inferred by minimizing the number of crossover events in each sibship.

To facilitate the multilocus linkage analysis, genotypes were processed to produce a maximum of five alleles at each marker locus while preserving linkage information. The most likely position of D19S841 with respect to the other markers was established within a subset of informative CADASIL families using the LINKMAP program. Then, the same program was run with all pedigrees to establish the best estimate of the CADASIL gene location. Due to computational limitations, two subintervals were analysed successively. Homogeneity was examined using the admixture test from the HOMOG program package (J. Ott 1991, Analysis of human genetic linkage, Johns Hopkins University Press, Baltimore, USA).

### RESULTS :

### Genetic homogeneity

Two-point linkage analysis data are shown on table 2. Significant Lod score (>3) were obtained in families F1, F5, F8, F12, F14 and F16 with several markers and positive Lod score were obtained in all other families strongly suggesting the genetic homogeneity of this condition. A maximum combined lod score of 37.24 was reached with marker D19S841 at θ = 0.01.

Admixture analysis of the two-point data with the HOMOG package showed evidence for linkage with homogeneity for all eight markers, with a significance level (P) of the χ² test <0.0001 for all of them. Admixture analysis of the multipoint data also supported a strong evidence for genetic homogeneity (P<0.0001) and estimated the proportion of linked families to be 1.0 (95% confidence interval 0.81-1.00).

### Refining of the CADASIL interval.

### High resolution genetic map of the D19S221-D19S215 interval:

The previous order of the polymorphic markers within the D19S221-D19S215 interval was the following : tel-D19S221-(D19S226/D19S411)-(D19S253/D19S244)-D19S199-D19S215-c en. The analysis of inherited haplotypes, both high-and low-risk haplotypes inherited from the affected parent and haplotypes inherited from the unaffected parent, revealed 6 cross-over events which led to refine the position of marker D19S411 as well as to precisely map marker D19S841 (Figure 1).

These 6 cross-over events, shown in Figure 3, strongly suggest that the most likely order of the markers is the following : tel-D19S221-D19S226-D19S841-(D19S253/D19S244/D19S411)-D19S199-cen.

The multipoint linkage analysis conducted in order to estimate the most likely position of the new marker D19S841 throughout the fixed map tel-D19S226-(0.01)-D19S253-(0.01)-D19S199-cen showed the following maximum-likehood order : tel-D19S226-(0.009)-D19S841-(0.001)-D19S253-(0.01)-D19S199-cen, odds against all alternatives being at least 10,000:1.

### Genetic mapping of the CADASIL locus between D19S226 and D19S199 :

Haplotype analysis in recombinant individuals established that the CADASIL locus is flanked proximally by marker D19S199 (3 recombination events : F1-24, F11-5 et F12-17) and distally by marker D19S226 (3 recombination events : F3-1, F8-7, F11-2). These recombination events were observed in 3 affected individuals (two clinically affected individuals having an abnormal MRI (F3-1, F11-2) and one asymptomatic individual with an obviously abnormal MRI (F1-24) and three healthy subjects with a normal MRI, aged above 40 (F8-7, F11-5 and F12-17) (Figure 4).

Two clinically asymptomatic subjects, F1-28 (27 years old) and F5-19 (40 years old), diagnosed as affected on the basis of the MRI, have inherited the low-risk haplotype from their affected parent. F1-28 is uninformative at three markers from the interval (D19S841, D19S253 and D19S411) and F5-19 is uninformative for D19S253. These two individuals could be double recombinants, but most likely are in fact missclassified, since their MRI showed only one small hypersignal which may be due to another cause. Another asymptomamic subjects F12-25, classified as unaffected, had inherited the high risk haplotype from her affected mother ; she is uninformative at D19S841 and D19S411. This individual, aged 42, may either be a double recombinant or represent a rare case of late onset of this disease. Except for these three individuals, no recombinant event was observed between D19S841, D19S253, D19S244, D19S411 and the CADASIL locus.

Multipoint analysis was used to best estimate the position of the CADASIL gene within two intervals : tel-D19S221-(0.06)-D19S226-(0.02)-D19S199-cen and tel-D19S226-(0.009)-D19S841-(0.011)-D19S199-cen. As shown in figure 2, the best estimate of the CADASIL gene location is between D19S226 and D19S199. The odds against the placement beyond D19S226 and D19S199 are, respectively, 209 : 1 and 3,715 : 1.

### Discussion :

The CADASIL locus was previously mapped in a 12 cM interval on chromosome 19 by a linkage analysis conducted on two pedigrees. Herein it is reported genetic linkage data obtained in 13 additional families. These data strongly suggest the genetic homogeneity of this condition and allowed to refine the mapping of the affected locus to a 2 cM interval bracketed by D19S226 and D19S199.

### Protocol of the diagnosis

The protocol which will be described hereinafter is given by way of example and as such is not considered as limitative. On the basis of the polymorphisms disclosed therein, all known methods of indirect genotypic diagnosis are applicable.

Protocol to select informative markers with respect to a family and/or to establish a diagnosis of a family :
- obtaining DNA from the available family members whose status with respect to the disease is known (e.g. with the MRI status) ; the nuclear DNA can for example be isolated from peripheral blood leucocytes, lymphoblastoid cell lines, cultured amniotic fluid cells, or chorionic villi, by standard proteinase K treatment and phenol-chlorophorm extraction techniques, and amplified or digested with the appropriate restriction enzymes if needed.
- amplifying the polymorphic alleles of the microsatellites by the PCR technique, using advantageously an automatic thermocycler apparatus such as the PHC3 Techne apparatus. See paragraph PCR hereinafter.
- analysing the size of the amplification products (amplimers), by electrophoresis on denaturating acrylamid DNA sequencing gel, blotting on Nylon membranes, hybridyzing with radiolabelled suitable repeats probes.
- alternatively, restriction polymorphic fragments can be analysed on agarose gels after Southern blotting.
- data obtained from autoradiographies are computed and Lod-score calculations are carried out using the M-LINK program.
- the assertion of linkage or absence of linkage is derived from statistical analysis, a Lod-score above 3 establishing unambiguous linkage of the disease gene to the tested marker.
- determination of the most informative polymorphims (microsatellites) for the studied family.

Protocol to diagnose an at risk individual from an affeted family :
- selecting informative markers by applying the above protocol to the family.
- obtaining DNA from the individual in the same manner than above.
- amplifying as above.
- analyzing the amplification products as above.
- using the linked polymorphic markers, a DNA based carrier risk can be calculated using the M-LINK program.

The same method can be used for prenatal carrier risk diagnosis.

**PCR**. Polymorphic genomic sequences were amplified by PCR using a PHC3 Techne apparatus. The reactions were performed in a final volume of 50 µl containing 200 ng of genomic DNA, 125 µM 4 dNTP mix, 1xPCR Boehringer Taq polymerase buffer, 1 U Boehringer Taq Polymerase, 1 µM of each primer (oligonucleotide sequences). Samples were processed through 30 temperature cycles (1st cycle, 94°C for 5 min ; 28 cycles including a denaturation step at 92°C for 1 min, an annealing step at 55°C for 1 min and an extension step at 72°C for 1 min ; the last cycle allowed extension at 72°C for 10 min).

After addition of 75 µl of loading buffer the samples were denatured for 10 min at 94°C, then laid on a 6% acrylamide DNA sequencing gel. After blotting, nylon membranes were fixed in 0.4 M sodium hydroxyde and hybridized with a (CA) 12mer, P32 labelled probe for 14 h.

**Linkage analysis.** Two point and multipoint linkage analysis were performed using the LINKAGE package.

## Claims

1. Method for the indirect genotypic diagnosis of CADASIL for symptomatic or at risk individuals or fetuses belonging to a family suspected or known to be affected by CADASIL, the method comprising the use of markers genetically linked to the mutated gene responsible for CADASIL in order to detect whether or not the tested individual is carrying the chromosome 19 markers alleles that has been linked to the disease gene in this given family and to estimate his carrier risk, characterized in that the method is based on the localization of the gene in the interval of 2 cM spanned by the flanking markers D19S226 and D19S199 and in that one uses at least two markers located each on one side of the gene.

2. Method according to claim 1, characterized in that one uses at least two markers selected from the group consisting of the flanking markers and the markers located within the 2 cM interval to obtain a diagnosis with a percentage of error risk estimated to be below 10⁻⁴.

3. Method according to claim 1, characterized in that one uses at least one marker selected from the group consisting of the following microsatellites : D19S226, D19S199, D19S841, 11547, D19S253 and D19S244.

4. Method according to claim 3, characterized is that one uses both flanking markers D19S226 and D19S199.

5. Method according to claim 1, characterized in that one uses three markers, say D19S226, D19S199 and one marker located within the 2 cM interval.

6. Method according to any one of claims 1 and 3 to 4, characterized in that one uses further at least one marker selected from the group consisting of the following microsatellites : D19S221, D19S179, D19S252, 19S415 and D19S215.

7. Method according to any one of claims 1 to 6, characterized in that one uses primer pairs which hybridize specifically on DNA sequences flanking the markers alleles known to be linked to the mutated gene responsible for CADASIL by ill individuals, one obtain DNA from the individual to be tested, one amplifies the DNA in the presence of the primer pairs and one analyzes the amplification products to assess the presence or the absence of allele markers linked to the mutated gene or the carrier risk of the tested individual with respect to these alleles markers.

8. Method according to claim 7, characterized in that one amplifies the DNA by Polymerase Chain Reaction.

9. Method according to claim 7 or 8, characterized in that one hybridizes the primer pairs on the DNA sequences at a temperature around 55°C.

10. Method according to any one of claims 7 to 9, characterized in that to analyze the amplification products one analyses their size.

11. Method according to claim 10, characterized in that one analyzes the size of the amplification products by electrophoresis on acrylamide denaturing gels.
